# EUROPEAN PATENT APPLICATION

(11) **EP 3 459 518 A1**
(43) Date of publication of application: **27.03.2019**
(21) Application number: 17461613.6
(22) Date of filing: 26.09.2017
(51) Int. Cl.: A61J 15/00, A61B 17/11, A61F 2/04, A61B 17/00

(54) **A FISTULA DEVICE**

(71) Applicant: Unipad Sp. z o.o., 60-476 Poznan (PL)
(72) Inventor: Banasiewicz, Tomasz, 62-005 Czerwonak (PL)
(74) Representative: Kancelaria Eupatent.pl Sp. z.o.o

(57) **Abstract**

A fistula device for fistula management, the device having a form of a T-pipe comprising: a bowel duct (11) having two free ends configured to be inserted into the intestine (20); and a discharge duct (12) having a first end connected to the bowel duct (11) and a second end terminated with an outlet opening (121) configured to extend outside the intestine (20); wherein the bowel duct (11) comprises an inlet section (11a) having an inlet opening (111) at the first end of the bowel duct (11) and an outlet section (11b) having an outlet opening (112) at the second end of the bowel duct (11), characterized in that the fistula device further comprises: a nutritional duct (13), for enteral feeding, extending along the discharge duct (12) and along the outlet section (11b) of the bowel duct (11).

## Description

### TECHNICAL FIELD

The present invention relates to a fistula device for fistula management during a treatment. The presented fistula device is designed to allow simultaneous enteral feeding of the patient and controlled collecting of the fistula secretion.

### BACKGROUND

A fistula is an abnormal connection between two hollow spaces in a body, such as between an external lumen and a gastrointestinal organ, between two gastrointestinal organs or between a gastrointestinal tract and a peritoneal cavity.

Fistulas of the gastrointestinal tract, including small intestine fistulas, may form as a result of surgical misadventures or various inflammatory bowel diseases, such as for example Crohn's disease, cancer, or various injuries.

Fistulas have been associated with considerable morbidity in patients. The complications of fistulas include water-electrolyte disturbances, infections, bleeding and multi-organ failure, which may lead even to the patient's death. These complications in patients with fistulas are due to the fistulas secretions which leak out through the fistula tract, whereas in the case of internal fistulas, such as the fistula between the gastrointestinal tract and the peritoneal cavity, the fistula secretion collects within the peritoneal cavity leading to limited or spilled peritonitis.

Typically, internal fistulas, including intestinal fistulas, are problematic in treatment, wherein most of the failures in fistulas treatment are caused by loss of liquids, electrolyte disturbances, multi-organ failures and patient's malnutrition.

In order to limit the loss of electrolytes and patient's malnutrition, parenteral nutrition is typically applied in patients with fistula since traditional or enteral feeding may not be suitable due to leaking out the nutrients through the fistula, which in turn negatively affects the patient's condition since the nutrients cannot be properly assimilated by the body.

There are known various devices for discharging the fistula secretion out of the body. Such devices are typically equipped with a duct, for example in a form of a tube, having two ends, wherein a first end is suitable for introduction into the intestine through the fistula, and a second end is arranged so as to extend out of the patient's body and is connected to a reservoir for fistula secretion, thereby enabling collection of the fistula secretion and preventing the secretion from leakage inside a peritoneal cavity.

There are known various devices suitable for collection of the fistula secretion out of the patient's body during fistula treatment.

A US patent application US20150297201 discloses a fistula management device for preventing enteric contents from leaking out of a bowel via a fistula opening in the bowel. The device comprises a tubular body that defines a main fluidic passageway extending from a first axial end to a second axial end of the tubular body. Inflatable cuffs are located at each of the first and second axial ends of the tubular body for creating a fluid seal between the first and second axial ends of the tubular body and inner walls of a bowel. However, such fistula management device is not suitable for simultaneous enteral feeding and removal of the fistula secretion.

A US patent application US20160325027 discloses a multi-lumen tubular device that can deliver wound irrigation, wound suction, and fistuloclysis nutritional support through the lumens of the tubular device. However, such device is not suitable for simultaneous enteral feeding and controlled removal of the fistula secretion outside the patient's body.

There is a need to provide a fistula device having a structure that enables simultaneous controlled removal of the fistula secretion and enteral feeding of a patient suffering from fistula, thereby providing increased chance for improvement of the patient's condition.

### SUMMARY

There is disclosed a fistula device for fistula management, the device having a form of a T-pipe comprising a bowel duct having two free ends configured to be inserted into the intestine; and a discharge duct having a first end connected to the bowel duct and a second end terminated with an outlet opening configured to extend outside the intestine; wherein the bowel duct comprises an inlet section having an inlet opening at the first end of the bowel duct and an outlet section having an outlet opening at the second end of the bowel duct. The fistula device further comprises a nutritional duct, for enteral feeding, extending along the discharge duct and along the outlet section of the bowel duct.

The nutritional duct may have a diameter smaller than a diameter of the bowel duct and smaller than a diameter of the discharge duct.

The nutritional duct may extend inside the bowel duct and inside the discharge duct, adjacently to their side walls.

The nutritional duct may have an inlet opening arranged at the free end of the discharge duct and has an outlet opening arranged at the outlet opening of the outlet section of the bowel duct.

The nutritional duct may have the inlet opening arranged on a stub tube protruding outwardly from the side wall of the discharge duct.

The inlet section of the bowel duct may have a conical shape with a diameter increasing towards the inlet opening.

The outlet section of the bowel duct may have a conical shape with a diameter increasing towards the outlet opening.

### BRIEF DESCRIPTION OF DRAWINGS

The object of the invention is presented by means of exemplary embodiments in a drawing, wherein:
Fig. 1 presents schematically a perspective view of the fistula device inserted in an intestine;
Fig. 2 presents schematically a cross-section of a first embodiment of the fistula device.

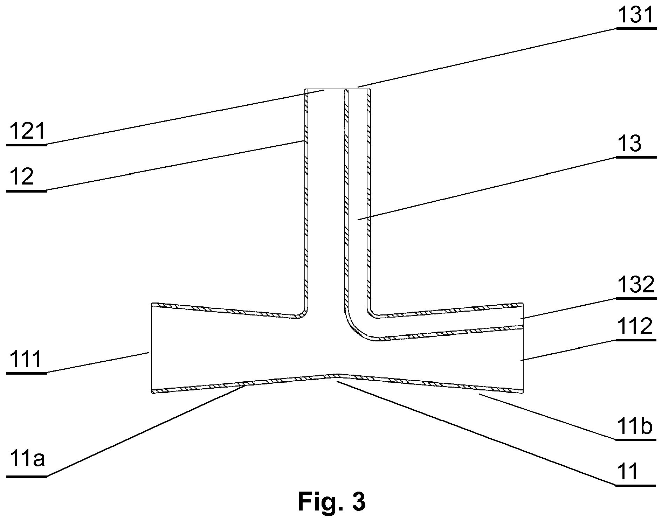

Fig. 3 presents schematically a cross-section of a second embodiment of the fistula device.

### DETAILED DESCRIPTION

A fistula device presented herein enables management of an intestinal fistula which, in particular, includes sealing of the fistula by inhibition of leakage of fistula content out of a gastrointestinal tract into a peritoneal cavity. Moreover, the structure of the fistula device provides simultaneous enteral nutrition and controlled collection of a fistula secretion outside of a patient's body - without disturbance to a healing process and/or therapy of the fistula, which further provides improvement in condition of water-electrolyte management of the body, progression in general health condition in patients as well as it increases chances for healing.

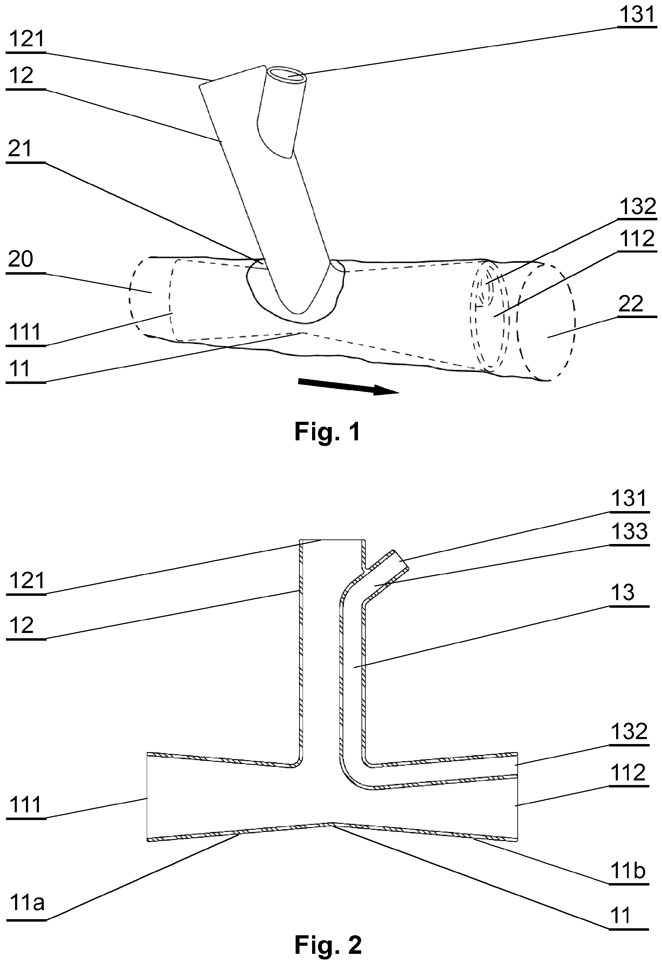

Fig. 1 shows schematically a fistula device installed in an intestine fistula 21 to enable fistula management. The fistula device has a form of a T-pipe. It comprises a bowel duct 11 for inserting into the intestine 20 close to the fistula 21. The fistula device further comprises a discharge duct 12 that projects out of a side wall of the bowel duct 11 and is throughly connected to the bowel duct 11. The discharge duct 12 defines an output passage for the fistula secretion and therefore it controls the collection of the fistula secretion outside the patient's body.

The bowel duct 11 has two free ends. It has an inlet section 11 a having an inlet opening 111 arranged on a first free end of the bowel duct 11 and an outlet section 11 b, arranged downstream the inlet section 11 a, and having an outlet opening 112 arranged on a second free end of the bowel duct 11. The inlet section 11 a comprises two outlets, wherein one outlet faces a lumen of the outlet section 11 b and the other outlet faces a lumen of the discharge duct 12. The outlet section 11 b comprise two inlets, wherein one inlet faces the lumen of the inlet section 11 a and the other inlet faces the lumen of the discharge duct 12.

The fistula device further comprises a nutritional duct 13 for enteral feeding, by transporting a nutritional composition from an outside of the patient's body into the intestine, through the nutritional duct 13 and therefore trough the fistula. Preferably, the nutritional duct 13 is an L-shaped tube provided along the discharge duct 12 and along the outlet section 12b of the bowel duct 11.

Preferably, the diameter of the nutritional duct 13 is smaller than the diameter of the bowel duct 11. Preferably, the diameter of the nutritional duct 13 is also smaller than the diameter of the discharge duct 12. Minimizing the diameter of the nutritional duct 13 allows to maximize the other, main ducts. The diameter of the nutritional duct 13 can be from 3 to 6 mm. The diameter of the discharge duct 13 depends on the fistula diameter, the channel shape and the technical requirements related to connecting the fistula device with the dressing, drains and bags, it can be from 6 to 16 mm. The diameter of the bowel duct 11 depends on the size of the lumen of the patient's intestine, it can be from 6 to 30 mm.

The bowel duct 11 has a shape of a funnel with a diameter extending towards its ends thereof, with a large end diameter, such as 30 mm. In case the diameter of the patient's intestine is smaller, the bowel duct 11 can be shortened to a position wherein its diameter corresponds to the intestine diameter - this makes the fistula device universal and suitable for insertion to intestines of various diameters.

Preferably, the nutritional duct 13 is provided inside the discharge duct 12 and inside the outlet section 11b of the bowel duct 11, on the side walls of these ducts. Such a compact structure facilities installation of the fistula device inside the intestine due to lack of protruding elements within the device structure. Furthermore, it provides a proper arrangement for all of the ducts of the fistula device inside the patient's body, even in case of patient's movement. The compact structure of the fistula device provides fixed arrangement of the device ducts, which in turn provides effective separation of the nutrition stream supplied into the body through the nutrition duct 13 from the fistula secretion discharged at the same time from the patient's intestine through the discharge duct 12.

The nutritional duct 13 has an outlet opening 132 arranged close to the outlet opening 112 of the outlet section 11 b of the bowel duct 11. Such arrangement of the outlet opening 132 of the nutritional duct 13 provides proper direction of administration of the nutritional content into the bowel, which is consistent with the peristaltic movement of the intestine.

The nutritional duct 13 has an inlet opening 131 arranged at the discharge duct 12.

The inlet opening 131 of the nutritional duct 13 may be arranged close to the outlet opening 121 of the discharge duct 12, as shown in Fig. 1.

Alternatively, as shown in Fig. 2, the inlet opening 131 of the nutritional duct 13 may be provided at the end of the stub tube 133 that protrudes out of the side wall of the discharge duct 12. In this configuration, the nutritional duct 13 is arranged inside the discharge duct 12 as well as inside the outlet section 11 b of the bowel duct 11 - at least within the portions of the fistula device that are located inside the intestine 20.

In order to install the fistula device inside the intestine 20, the bowel duct 11 is introduced into the intestine 20 through the fistula 21 and positioned coaxially with respect to the longitudinal axis of the intestine 20, so that the outlet opening 112 of the outlet section 11 b is arranged in a direction consistent with the peristaltic movement of the intestine 22, and the inlet opening 111 of the inlet section 11 a is arranged in a direction opposite to the peristaltic movement of the intestine 22. The direction of the peristaltic movement of the intestine is shown schematically in Fig 1 by an arrow 22. Such arrangement of the fistula device within the intestine 20 provides simultaneous stemming of the uncontrolled leakage of the fistula secretion, from the intestine into the peritoneal cavity, and unaffected transport of the intestinal content through the intestine 22. If the external diameter of bowel duct 11 is equal to the internal diameter of the intestine 20, then the fistula device may stabilize within the intestine by itself.

After the fistula device is installed in the intestine 20 at the fistula tract, the outlet opening 121 of the discharge duct 12 may be connected to an external system for receiving the fistulas secretions, whereas the inlet opening 131 of the nutritional duct 13 may be connected to a system for administration of enteral feeding.

During the ongoing digestive processes within the digestive tract, the fistula secretion is collected in the inlet section 11 a of the bowel duct 11, from where the secretion moves into the discharge duct 12, whereas the secretion is safely removed out of the patient's body via the discharge duct. Additionally, during the removal of the fistula secretion, nutrients may be introduced via the nutritional duct 13 into the patient's intestine - in a close proximity to the outlet passage 112 of the bowel duct 11.

Moreover, due to the open passage structure of the bowel duct 11, the fistula device provides efficient transport of the intestinal content through the inlet 11 a and outlet 11 b section of the bowel duct 11, thereby supporting the natural transport of the intestinal content in the direction consistent with the peristaltic movement.

In a preferred embodiment, the inlet section 11 a of the bowel duct 11 may have a conical shape with its diameter increasing towards the inlet opening 111 of the inlet section 11 a. Moreover, the outlet section 11 b of the bowel duct 11 may have a conical shape with its diameter increasing towards the outlet opening 112 of the outlet section 11 b. The conical shape of the inlet section 11 a and/or the outlet section 11 b of the bowel duct 11 allows the diameter of respective section 11 a, 11 b to be quickly and easily adjusted by shortening the length of the respective section 11 a, 11 b at the site of the inlet 111 and/or outlet 112 opening, respectively. This provides simple adjustment of the diameter of the bowel duct 11 to the diameter of the patient's intestine, while preserving a simple structure of the fistula device.

The structure of the fistula device enables simultaneous treatment and/or therapy of a fistula and enteral feeding of a patient via the fistula. The nutritional duct 13 may be used during the removal of the fistula secretion via the inlet section 11 a of the bowel duct 11 and the discharge duct 12, without the risk of infection of the nutrients present in the nutritional duct 13.

Moreover, the outlet opening 121 of the discharge duct 12 may be terminated with a conventional ending, such as for example the ending in the Foley's catheter configured for connection with urine bags or drains. Furthermore, the outlet opening 121 of the discharge duct 12 may be terminated with an ending configured to be connected with various systems, such as negative-pressure wound therapy systems, simple or complex fluids collection systems, stoma bags, or poor man's vacuum systems.

Along with the enteral feeding, various methods of treatment or therapy of fistula may be applied in patients by using the fistula device due to the structure of the device that provides adequate protection of the fistula from contact with the intestinal-fistula secretions and access to the fistula secretion, inter alia, in order to remove the fistula secretion and to conduct further possible biochemical or microbiological examinations.

Moreover, after installation of the fistula device within the intestine, at the site of fistula tract, the fistula device provides simultaneous conducting of the enteral feeding and healing or therapy of the fistula, without disturbing of the ongoing healing process, which increases the chances of cure of the patient.

## Claims

1. A fistula device for fistula management, the device having a form of a T-pipe comprising:
- a bowel duct (11) having two free ends configured to be inserted into the intestine (20); and
- a discharge duct (12) having a first end connected to the bowel duct (11) and a second end terminated with an outlet opening (121) configured to extend outside the intestine (20);
- wherein the bowel duct (11) comprises an inlet section (11a) having an inlet opening (111) at the first end of the bowel duct (11) and an outlet section (11 b) having an outlet opening (112) at the second end of the bowel duct (11), **characterized in that** the fistula device further comprises:
- a nutritional duct (13), for enteral feeding, extending along the discharge duct (12) and along the outlet section (11 b) of the bowel duct (11).

2. The device according to claim 1, wherein the nutritional duct (13) has a diameter smaller than a diameter of the bowel duct (11) and smaller than a diameter of the discharge duct (12).

3. The device according to claim 2, wherein the nutritional duct (13) extends inside the bowel duct (11) and inside the discharge duct (12), adjacently to their side walls.

4. The device according to any of preceding claims, wherein the nutritional duct (13) has an inlet opening (131) arranged at the free end of the discharge duct (12) and has an outlet opening (132) arranged at the outlet opening (112) of the outlet section (11 b) of the bowel duct (11).

5. The device according to claim 4, wherein the nutritional duct (13) has the inlet opening (131) arranged on a stub tube (133) protruding outwardly from the side wall of the discharge duct (12).

6. The device according to any of preceding claims. wherein the inlet section (11 a) of the bowel duct (11) has a conical shape with a diameter increasing towards the inlet opening (111).

7. The device according to any of preceding claims, wherein the outlet section (11 b) of the bowel duct (11) has a conical shape with a diameter increasing towards the outlet opening (112).
